# EUROPEAN PATENT APPLICATION

(11) **EP 4 011 446 A1**
(43) Date of publication of application: **15.06.2022**
(21) Application number: 21202527.4
(22) Date of filing: 13.10.2021
(51) Int. Cl.: A61P 17/00, C08B 37/08

(54) **METHOD FOR PRODUCING A CROSS-LINKED HYALURONIC ACID HAVING THE ABILITY TO MODULATE THE RELEASE OF AMINO ACIDS USEFUL FOR THE BIOSTIMULATION OF COLLAGEN AND PRODUCT THUS OBTAINED**

(30) Priority: 21.10.2020 IT 202000024877
(71) Applicant: Italfarmacia S.r.l., 00155 Roma (IT)
(72) Inventor: MARCHETTI, Mario, 00155 Rome (RM) (IT); MARCHETTI, Marco, 00133 Rome (RM) (IT); MARCHETTI, Massimiliano, 06061 Castiglion del Lago (PG) (IT); CAVALLO, Giovanni, 00122 Ostia (RM) (IT); DE LORENZO, Antonino, 00133 Rome (RM) (IT); BURATTINI, Cristian, 00072 Ariccia (RM) (IT); DI RENZO, Laura, 00133 Rome (RM) (IT)
(74) Representative: Sarpi, Maurizio

(57) **Abstract**

A method for producing a cross-linked hyaluronic acid in the form of an injectable gel, sterile by sterilization at 121°C, wherein two amino acids such as glycine and proline are adsorbed, having the ability to modulate the release thereof, allowing a stimulation of collagen synthesis for a long time, includes the following steps:
I) a 10% non-linked hyaluronic acid is solubilized in a 1 N NaOH solution;
the polymerizing agent BDDE is added at 1% by weight with respect to the hyaluronic acid, and after homogenization and incubation at a controlled temperature,
the homogenized gel is neutralized and incubated with a 1% by weight phosphoric acid solution;

II) a phosphate buffer solution containing glycine and proline is added to the neutralized gel in an amount such that the final concentration of each of the two amino acids in the 2.4% cross-linked hyaluronic acid gel is between 1% and 2.5% by weight.
III) the adsorption of said amino acids is carried out in the cross-linked gel by swelling it for 7 days at room temperature under slight mixing until a compact and fluid gel is obtained where the previously added buffer solution is completely adsorbed; and
IV) the gel obtained is sterilized, divided into glass syringes, through sterilization at 121°C for 15 minutes.

## Description

### SUMMARY OF THE INVENTION

The present invention relates to a new cross-linked hyaluronic acid in the form of an injectable gel containing, with a specific adsorption procedure, two amino acids such as glycine and proline, useful for the synthesis of collagen.

The invention further relates to the method for producing injectable cross-linked hyaluronic acid as indicated above, having the ability to modulate the release of said adsorbed amino acids, allowing a stimulation of collagen synthesis for a long time, characterized in that the amino acids are added to the hyaluronic acid gel which is already fully cross-linked and therefore they do not participate in the cross-linking process in any manner.

### SCOPE OF THE INVENTION

In recent years, hyaluronic acid has been increasingly discussed as one of the most popular methods for treatments against skin aging. The use thereof is counted among the least invasive aesthetic techniques for reducing wrinkles and the formation thereof.

The technique through which this substance is inserted below the skin is that of fillers, which can have good and long-lasting results especially if cross-linked hyaluronic acid is used.

Hyaluronic acid is a substance naturally contained in our body. The purpose thereof is to regulate dermis hydration and volume. Furthermore, one of the functions thereof within the body is also to repair wounds and renew the skin.

Depending on the type of treatment and the composition of the acid, two categories can be distinguished: linear hyaluronic acid and cross-linked hyaluronic acid.

The first one - linear hyaluronic acid - lasts only a few weeks and is administered, above all, as a bio-revitalizing agent through a series of mini-injections.

The second one - cross-linked hyaluronic acid - progressively diffuses instead into the dermis and thus offers more lasting results on both wrinkles and the volume increase.

In order to switch from linear to cross-linked hyaluronic acid, it is necessary to perform a chemical lab procedure using either BDDE (Butanediol-diglycidyl ether) or other polymerizing agents.

The polymerization reaction with BDDE or other polymerizing agents makes the hyaluronic acid molecule more resistant to the degradation action of some enzymes, in particular the enzyme hyaluronidase which degrades hyaluronic acid into smaller fragments over time, thus reducing the residence thereof in the dermis.

The above statements demonstrate how the (linear and cross-linked) hyaluronic acid has a key use for eliminating and at least reducing facial imperfections.

In recent years, the use of hyaluronic acid in dermis biorevitalization/biostimulation has been added to this original application. The dermis is the connective tissue of the epidermis, responsible for maintaining the support and hydration thereof. The dermis has two very important proteins for the skin, known as collagen and elastin.

These two proteins are organized into fibers, which in turn intercalate with each other to form a real structure to support the epidermis, which thus acquires features such as compactness, elasticity, and tone.

Natural collagen is the most important structural protein in the body. It is the most abundant in mammals and has a finely controlled molecular production system. Among all the proteins, it is the most present in the human body. This substance therefore plays a key role for skin, cartilage, bones, tendons, membranes and blood vessels.

It is a simple protein, mainly formed by the association of four amino acids which are cyclically repeated in the peptide structure: glycine, proline, hydroxyproline and hydroxylysine, elements which favor the re-synthesis thereof in the body when deficient.

Unfortunately, as the body ages, it produces less and less collagen, and there are also external factors which compromise the quality and integrity thereof, such as UV rays.

All of this results in a weaker structure for the skin, which then pits and folds, forming wrinkles.

Patent documents WO 2019/130359, EP 3 666 278, WO 2011/148116, and the articles by Arora Gulhima et al. "Biorevitalization of the skin with skin boosters: Concepts, variables, and limitation", JOURNAL OF COSMETIC DERMATOLOGY, 10 August 2020 (2020-08-10) and by Diwan Zoya et Al.: "A Prospective Study on Safety, Complications and Satisfaction Analysis for Tear Trough Rejuvenation Using Hyaluronic Acid Dermal Fillers", PLASTIC AND RECONSTRUCTIVE SURGERY GLOBAL OPEN, Vol.8, no.4, April 2020 (2020-04-01) page e2753, describe both neutralization and mixing procedures to obtain cross-linked hyaluronic acid with PEGS, and methods are described for preparing cross-linked hyaluronic acid comprising lysine/or proline where said amino acids participate in the cross-linking reaction or form covalent bonds with the hyaluronic acid structure.

### TASK OF THE INVENTION:

Taking into consideration the above statements, the Applicant has developed a method for obtaining a cross-linked hyaluronic acid filler where, unlike what is known, the two amino acids glycine and proline, useful for stimulating collagen synthesis, are added to the hyaluronic acid gel already fully cross-linked with BDDE so that such amino acids do not participate in the cross-linking process in any manner.

Therefore, unlike the known art, the whole procedure for preparing cross-linked hyaluronic acid has been studied so that the cross-linked hyaluronic acid becomes a kind of reservoir of the two amino acids to be injected below the skin; summarily, a prolonged release of amino acids can be achieved over time, allowing a stimulation of collagen synthesis for a long time.

This injectable gel proved to provide a natural and regular improvement with considerable residence times, improving the stability of the cross-link and allowing elasticity and firmness of the system.

Further advantages and features of the invention will become apparent from the following detailed description and the examples shown with reference to the accompanying drawings, in which:
fig. 1 shows the apparatus used for a dialysis experiment to define the prolonged release of the amino acids glycine and proline from a cross-linked hyaluronic acid gel;
fig. 2 is a graph of the comparison of the glycine content (2.5%) in a hyaluronic acid gel (2.4%) and in a cross-linked hyaluronic acid gel (2.4%);
fig. 3 is a graph of the comparison of the% glycine release from a hyaluronic acid gel (2.4%) and a cross-linked hyaluronic acid gel (2.4%), both sterilized at 121°C;
fig. 4 is a graph of the comparison of the 2.5% proline content in a 2.4% hyaluronic acid gel and a 2.4% cross-linked hyaluronic acid gel, both sterilized at 121°C;
fig. 5 is a graph of the comparison of % release of the 2.5% proline from the 2.4% hyaluronic acid gel and the 2.4% cross-linked hyaluronic acid gel;
fig. 6 is a graph of the comparison of the glycine release from the cross-linked hyaluronic acid gel before and after sterilization at 121°C;
fig. 7 is a graph showing the decrease over time in the concentration of the amino acids glycine and proline subjected to dialysis in a buffer solution;
fig. 8 is a graph of the comparison of the (1%) glycine content over time from a 2.4% hyaluronic acid gel and a 2.4% cross-linked hyaluronic acid gel, both sterilized at 121°C;
fig. 9 is a graph of the comparison of the % glycine release over time from a hyaluronic acid gel (2.4%) and a cross-linked hyaluronic acid gel (2.4%), both sterilized at 121°C;

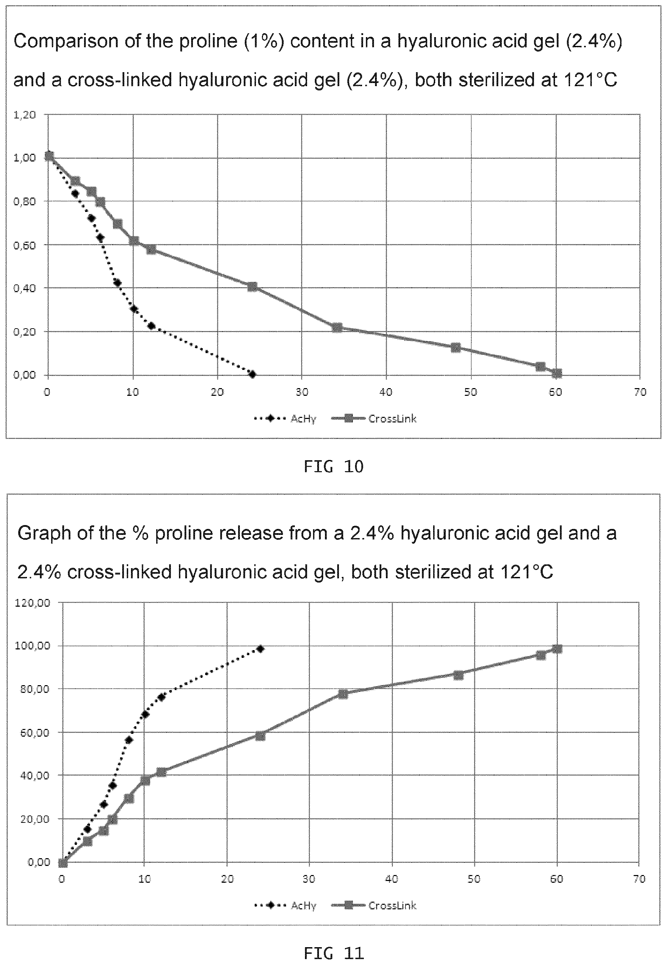

fig. 10 is a graph of the comparison of the (1%) proline content over time in a hyaluronic acid gel (2.4%) and a cross-linked hyaluronic acid gel (2.4%), both sterilized at 121°C;
fig. 11 is a graph of % proline release over time from a 2.4% hyaluronic acid gel and a 2.4% cross-linked hyaluronic acid gel, both sterilized at 121°C;

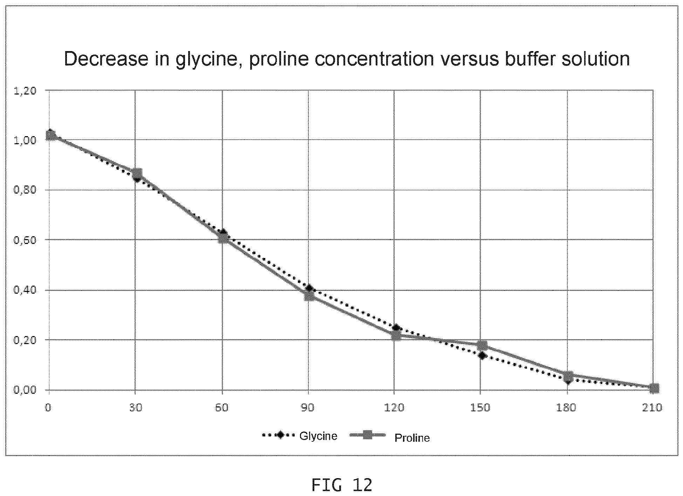

fig. 12 is a graph showing the decrease in the concentration of glycine and proline versus buffer solution.

### DETAILED DESCRIPTION OF THE INVENTION

We now describe, step by step, the preparation procedure, according to the present invention, of a cross-linked hyaluronic acid in which, after the completion of the cross-linking reaction, the two amino acids glycine and proline are adsorbed, useful for stimulating the synthesis of collagen, with the ability to modulate the release thereof.

The procedure includes the following steps:
A) Complete cross-linking of hyaluronic acid
B) Adsorption of amino acids in the cross-linked hyaluronic acid.

### A) Description of the method for cross-linking hyaluronic acid

The following three steps are included:

### Step 1: polymerization reaction

The hyaluronic acid (10%) is carefully solubilized in a 1.0 N Na0H solution;
The polymerizing agent, BDDE, 1% with respect to the weight of the hyaluronic acid is added;
The product is homogenized.

### Step 2: Controlled temperature incubation

Put the gel in the oven for 4 hours at 40°C.

### Step 3: Neutralization reaction

Neutralize the gel with a 1% phosphoric acid solution;

### B)- Description of the amino acid adsorption procedure in the cross-linked hyaluronic acid

### Step 1: Addition of amino acids

A phosphate buffer solution containing glycine and proline is added to the previously neutralized and cross-linked hyaluronic acid gel;
The final concentration of the two amino acids is 2.5% (25 mg/ml) and that of the hyaluronic acid is 2.4% (24 mg/ml).

### Step 2: Adsorption of amino acids in the cross-linked hyaluronic acid gel

The gel is left to swell for 7 days at room temperature under light mixing obtained by rotating the reaction container on a roller mixer;
after 7 days of mixing the gel is perfectly compact and fluid, the previously added buffer solution containing the two amino acids glycine and proline is completely adsorbed by the gel;

### Step 3: - Sterilization of the cross-linked hyaluronic acid gel containing the amino acids

The cross-linked hyaluronic acid gel with the adsorbed amino acids is divided into glass syringes (1.1 g each) and the syringes are sterilized at 121°C for 15 minutes at a pressure of 1 atm.

The evaluation of the prolonged release over time of the amino acids glycine and proline from the cross-linked hyaluronic acid gel thus obtained was defined by means of a dialysis experiment versus an isotonic phosphate buffer carried out by comparing the release time kinetics of the adsorbed amino acids within a cross-linked hyaluronic acid and the release of the amino acids simply solubilized in a linear hyaluronic acid gel, where the concentration of the hyaluronic acid is the same.

In the first case there is a macro structure of cross-linked hyaluronic acid similar to a porous sponge in which the amino acids diffuse, in a well-defined time and not immediately, in the second case the amino acids are simply solutes dispersed among the free hyaluronic acid molecules.

This peculiar feature, despite the simplicity thereof, leads to different amino acid release kinetics, whereby that referring to the cross-linked hyaluronic acid is longer than that of the free hyaluronic acid.

The following modes were followed:

### Dialysis procedure to define the prolonged release of the amino acids glycine and proline over time.

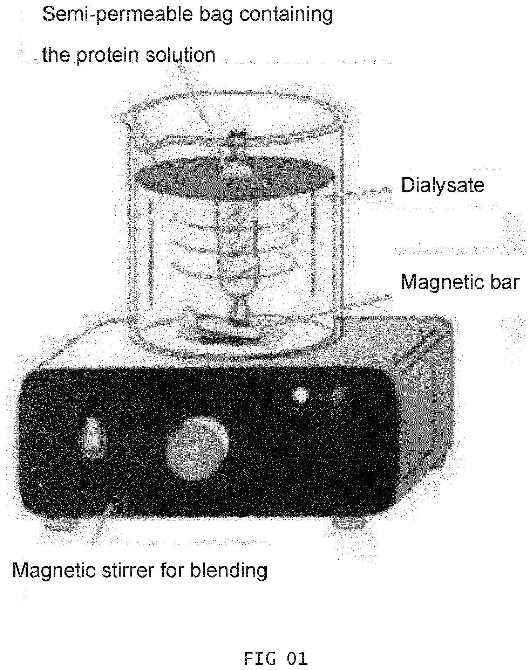

The apparatus used is illustrated in fig. 1.

With reference to such a figure 1, the dialysis experiment is briefly described:
1 gram of cross-linked hyaluronic acid gel (24mg/ml) vf containing 2.5% of each amino acid glycine and proline is placed in a Cut off 20KDalton dialysis tube 6.3 mm in diameter. The dialysis tube containing the cross-linked hyaluronic acid with proline and glycine, suitably closed at both ends, is weighed and put in dialysis against 40 ml of isotonic phosphate buffer solution, in a 100 ml Pyrex glass container, under magnetic stirring at constant speed.

The identification of a procedure for producing a cross-linked hyaluronic acid with an ability to modulate the release of amino acids useful for the bio-stimulation of collagen includes the following:

### Determination of the amino acid concentration

Aliquots of 0.1 ml are withdrawn at regular intervals and the concentration of glycine and proline is measured.

To properly evaluate the release profile of the amino acids glycine and proline specifically adsorbed in the cross-linked hyaluronic acid, a comparison was made between the release of the amino acids released by the 2.4% cross-linked hyaluronic acid gel and the release of same amino acids released by a 2.4% linear hyaluronic acid gel. The procedure followed is below:

### Step 1: preparation of a 2.4% hyaluronic acid gel

A 2.4% hyaluronic acid gel is prepared with the addition of glycine and proline at the final concentration of each amino acid of 2.5%.

The gel obtained is divided into glass syringes (1.1 g each) which are sterilized at 121°C for 15 minutes at a pressure of 1 atm.

### Step 2: Gel dialysis

1 gram of the gel is subjected to dialysis with the same procedure described above in point C).

The release of the amino acids of the two hyaluronic acids, one cross-linked and the other linear, is still followed in the manner indicated above.

### RESULTS

During the experiments carried out, the release of the two amino acids glycine and proline was obtained by plotting the concentration of amino acids as a function of time.

In order to better assess the adsorption capacity of the cross-linked hyaluronic acid, the amino acid concentration was decreased to 1%.

### 1st EXPERIMENT: Glycine 2.5%, Proline 2.5%

The same comparisons are carried out first for glycine and then for proline at the same concentration, under the same conditions.

### FOR GLYCINE:

The content over time of glycine (2.5%) in a hyaluronic acid gel (2.4%) and in a cross-linked hyaluronic acid gel (2.4%) are compared.

**TABLE 1**

| Time | | |
|---|---|---|
| hours | AcHy | CrossLinK |
| 0 | 2.52 | 2.52 |
| 3 | 2.25 | 2.41 |
| 6 | 1.86 | 2.13 |
| 8 | 1.46 | 1.88 |
| 10 | 1.26 | 1.54 |
| 12 | 1.06 | 1.4 |
| 24 | 0.05 | 0.64 |
| 30 | 0.01 | 0.52 |
| 36 | | 0.41 |
| 48 | | 0.33 |
| 50 | | 0.23 |
| 60 | | 0.01 |

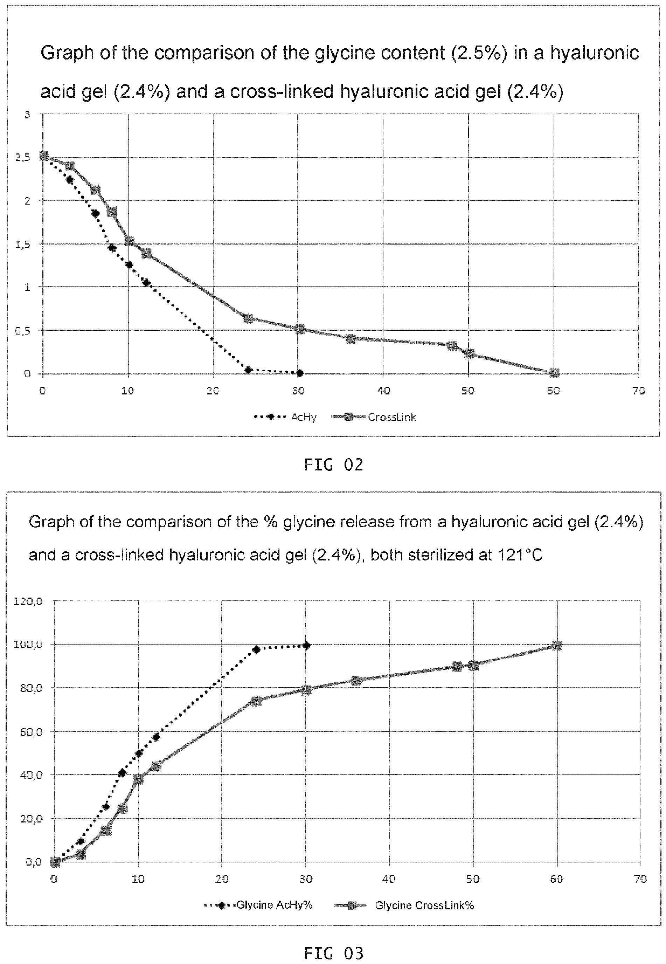

The data are shown in the graph in fig. 2, which indicates that the glycine content (2.5%) is depleted:
in 60 hours for cross-linked hyaluronic acid; (continuous line)
in 30 hours for hyaluronic acid: (dotted line)

The experiment was then repeated to compare the percentage release of glycine from a hyaluronic acid gel (2.4%) and from a cross-linked hyaluronic acid gel (2.4%), after carrying out sterilization at 121°C.

The relative data are shown in the following table:

**TABLE 2**

| time | AcHyal | CrossLink |
|---|---|---|
| hours | | |
| 0 | 0 | 0 |
| 3 | 12.4 | 3.6 |
| 6 | 25.6 | 16.4 |
| 8 | 38.8 | 24.8 |
| 10 | 48.8 | 37.5 |
| 12 | 55.2 | 43 |
| 24 | 94 | 68.8 |
| 30 | 97.2 | 75,5 |
| 36 | 99.6 | 83.3 |
| 48 | | 88.8 |
| 50 | | 91.2 |
| 60 | | 99.6 |

The graph obtained is shown in fig. 3, which indicates that the release of glycine (2.5) occurs:
in 60 hours for cross-linked hyaluronic acid (continuous line)
in 30 hours for hyaluronic acid (dotted line).

### FOR PROLINE:

The 2.5% proline content is compared in a 2.4% hyaluronic acid gel and in a 2.4% cross-linked hyaluronic acid gel.

The data obtained are shown in the following Table 3:

**TABLE 3**

| time | AcHyal | CrossLink |
|---|---|---|
| hours | | |
| 0 | 0 | 0 |
| 3 | 12.4 | 3.6 |
| 6 | 25.6 | 16.4 |
| 8 | 38.8 | 24.8 |
| 10 | 48.8 | 37.5 |
| 12 | 55.2 | 43 |
| 24 | 94 | 68.8 |
| 30 | 97.2 | 75,5 |
| 36 | 99.6 | 83.3 |
| 48 | | 88.8 |
| 50 | | 91.2 |
| 60 | | 99.6 |

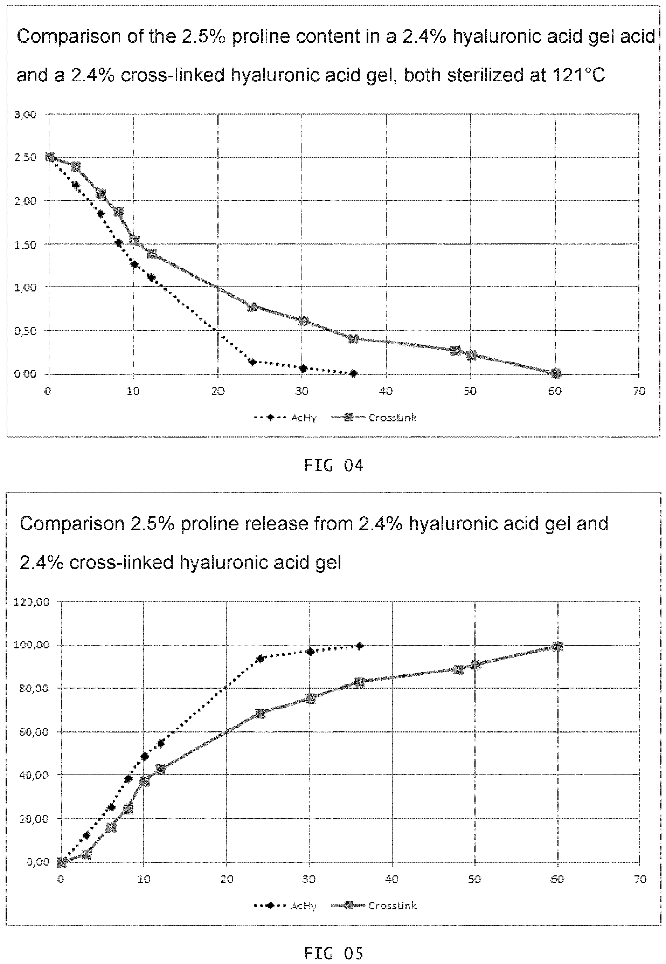

The graph obtained is shown in fig. 4 and indicates that the release of proline (2.5%) occurs:
- in 60 hours for cross-linked hyaluronic acid (continuous line)
- in 36 hours for hyaluronic acid (dotted line)

The experiment is then repeated to compare the 2.5% proline content in a 2.4% hyaluronic acid gel and a 2.4% cross-linked hyaluronic acid gel, both sterilized at 121°C.

The data obtained are shown in the following Table 4.

**TABLE 4**

| time | AcHyal | CrossLink |
|---|---|---|
| hours | blue | red |
| | | |
| 0 | 2.51 | 2.51 |
| 3 | 2.19 | 2.41 |
| 6 | 1.86 | 2.09 |
| 8 | 1.53 | 1.88 |
| 10 | 1.28 | 1.55 |
| 12 | 1.12 | 1.4 |
| 24 | 0.15 | 0.78 |
| 30 | 0.07 | 0.62 |
| 36 | 0.01 | 0.41 |
| 48 | | 0.28 |
| 50 | | 0.22 |
| 60 | | 0.01 |

The graph obtained is shown in fig. 5 and indicates that the proline content (2.5%) is depleted:
- in 60 hours for cross-linked hyaluronic acid (continuous line)
- in 36 hours for hyaluronic acid (dotted line)

A study of the release of glycine on the cross-linked hyaluronic acid gel was then carried out before and after sterilization at 121°C.

The data collected are shown in the following Table 5.

**TABLE 5**

| time | sterile | not sterile |
|---|---|---|
| hours | | |
| 0 | 2.52 | 2.52 |
| 3 | 2.41 | 2.45 |
| 6 | 2.13 | 2.2 |
| 8 | 1.88 | 1.91 |
| 10 | 1.54 | 1.57 |
| 12 | 1.22 | 1.35 |
| 24 | 0.64 | 0.75 |
| 30 | 0.52 | 0.64 |
| 36 | 0.41 | 0.45 |
| 48 | 0.33 | 0.34 |
| 50 | 0.23 | 0.25 |
| 60 | 0.01 | 0.0 1 |

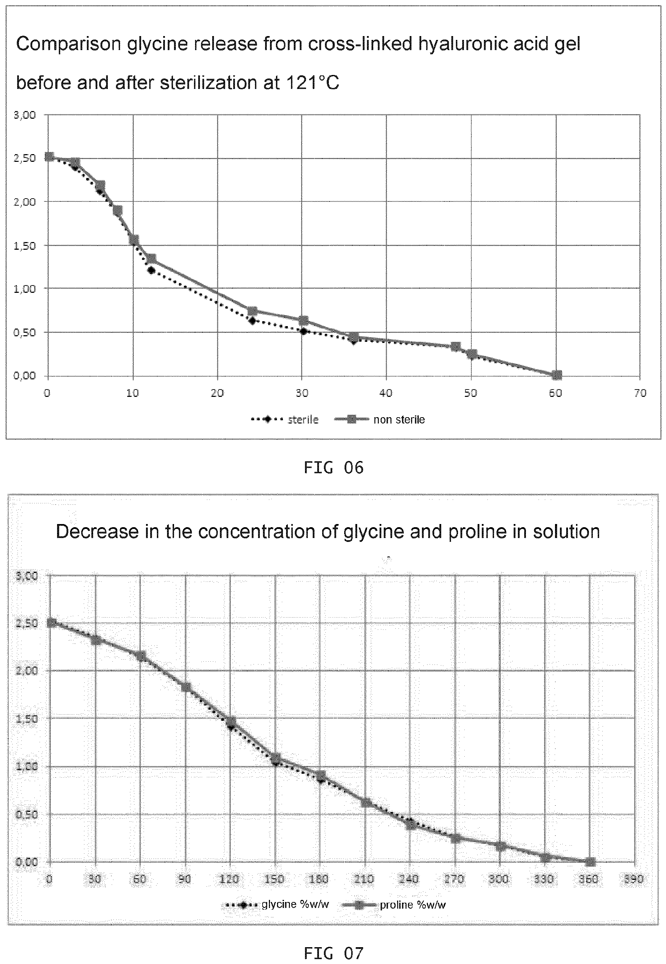

The graph obtained is shown in fig. 6 and shows how the sterilization of cross-linked hyaluronic acid at 121°C does not substantially modify the release of glycine, showing a good structural stability of the product, as compared to the same non-sterilized product.

### Verification of the decrease in the concentration of amino acids subjected to dialysis versus a buffer solution.

The data obtained are shown in the following Table 6.

**TABLE 6**

| time | glycin e | proline |
|---|---|---|
| minut es | % w/w | % w/w |
| 0 | 2.52 | 2.51 |
| 30 | 2.35 | 2.33 |
| 60 | 2.15 | 2.17 |
| 90 | 1.83 | 1.84 |
| 120 | 1.42 | 1.48 |
| 150 | 1.05 | 1.1 |
| 180 | 0.87 | 0.92 |
| 210 | 0.64 | 0.63 |
| 240 | 0.43 | 0.39 |
| 270 | 0.26 | 0.25 |
| 300 | 0.17 | 0.18 |
| 330 | 0.05 | 0.06 |
| 360 | 0.01 | 0.01 |

The graph obtained is shown in fig. 7 and indicates that the release of both amino acids at a concentration of 2.5% occurs in 360 minutes (6 hours).

### 2nd EXPERIMENT: Glycine 1%. Proline 1%

To better assess the adsorption capacity of the cross-linked hyaluronic acid, the amino acid concentration was decreased to 1%.

A comparison of the glycine content (1%) was therefore made from a 2.4% hyaluronic acid gel and a 2.4% cross-linked hyaluronic acid gel, both sterilized at 121°C.

The data obtained are shown in the following table 7.

**TABLE 7**

| | **Ac Hy** | **Cross** |
|---|---|---|
| time | Glycine | Glycine |
| hours | with % | conc |
| 0 | 1.01 | 1.01 |
| 3 | 0.82 | 0.89 |
| 5 | 0.7 | 0.82 |
| 6 | 0.63 | 0.79 |
| 8 | 0.41 | 0.74 |
| 10 | 0.33 | 0.65 |
| 12 | 0.21 | 0.6 |
| 24 | 0.01 | 0.39 |
| 34 | | 0.25 |
| 48 | | 0.11 |
| 58 | | 0.06 |
| 60 | | 0.01 |

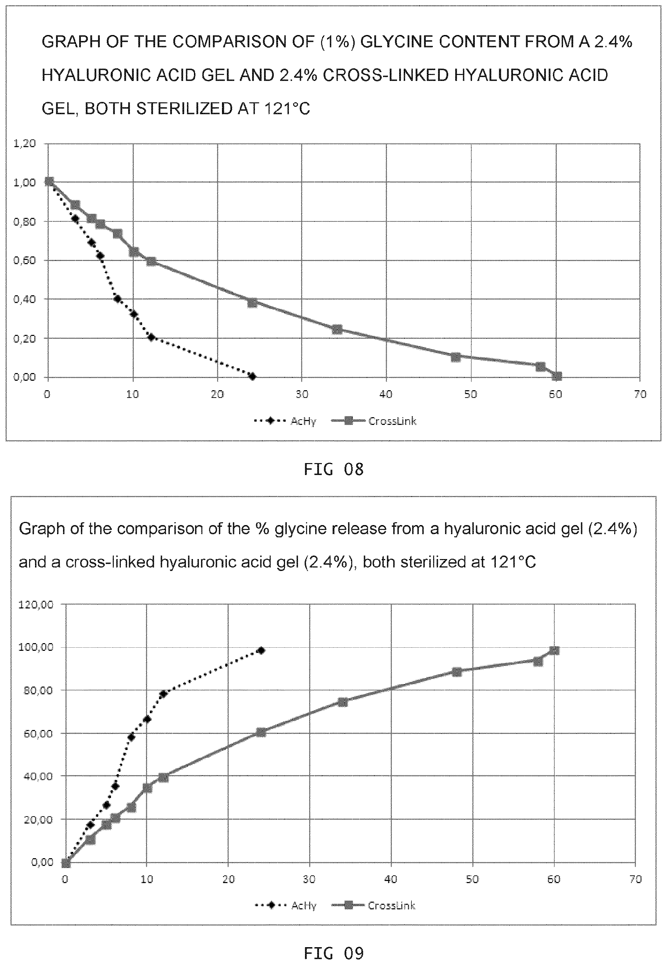

The graph obtained is shown in fig. 8 and indicates that the release of glycine (1%) occurs:
- in 60 hours for cross-linked hyaluronic acid (continuous line)
- in 24 hours for hyaluronic acid (dotted line).

### CONCLUSION

The experiments carried out show that the cross-linked hyaluronic acid synthesis procedure and the subsequent amino acid adsorption step have transformed the cross-linked hyaluronic acid into a "reservoir" of amino acids with the ability to release them over a longer time than that of a linear hyaluronic acid gel at the same concentration.

Furthermore, the experiments carried out have shown that surprisingly the release over time of the two amino acids glycine and proline is not substantially modified, switching from the concentration of 2.5% to the concentration of 1%, with the same concentration of 2.4% cross-linked hyaluronic acid.

Considering the different concentrations, a longer release over time could have been expected in the case of the 2.5% concentration, compared to that of 1%. Simply put, the more amino acid adsorbed, the longer the release should be. If this does not occur, it must be inferred that there is a precise relationship between the amount of cross-linked hyaluronic acid (a certain number of pores, interstices), and the amount of amino acid adsorbed. This peculiarity is apparently related to the particular cross-linking procedure with BDDE, the relationships between the various constituents, the time and temperature of the cross-linking reaction, combined in particular with the adsorption time and temperature.

### CLINICAL RESULTS.

The 2.4% cross-linked hyaluronic acid hydrogel, in which glycine and proline (2.5%) are absorbed with the described procedure and for which the prolonged release capacity thereof has been evaluated over time, was subjected to an open study to evaluate the performance thereof in correcting aesthetic facial defects.

The treatments were proposed to male and female subjects aged between 30 and 70 years with follow-up for defects in the aforesaid facial areas with evaluation of the efficacy on volumes (facial volume loss scale FVLS) and on wrinkles (using the wrinkle severity rating scale WSRS) at 14 days and at 1, 3, 6, 9 and 12 months with respect to the baseline.

The assessment of aesthetic results included skin hydration, image analysis of nasolabial folds, zygomatic volumes (3D), and photographic documentation. Furthermore, keeping informed consent and the necessary standards of hygiene, safety, product management (extrusion from the syringe, distribution of the injection in the tissue), as well as softness to the touch, the satisfaction of the patient and operating doctor were evaluated, including post-session satisfaction, and pain tolerance.

### RESULT ANALYSIS:

The hydrogel proved to be fluid in filling, while maintaining the compactness of the injection, which can be modeled both by the operator and by mimic movements, conferring a volumetric increase with natural-looking tissue penetration and with a statistically significant aesthetic result both immediately and at distance of 6 months and 8 months from the first injection.

Good results were also obtained during the study on the degree of skin hydration and the revitalizing effect in the tissue, with evidence on the consistency (texture) and brightness of the skin. There were no serious treatment-related adverse events.

### FINAL CONSIDERATIONS:

This injectable gel proved to provide a natural and regular improvement of aesthetic facial defects with considerable residence times, improving the stability of the cross-link, allowing elasticity and firmness of the system. It received the approval of patients and operators.

Good results were achieved on hydration and skin texture during the study, whereby both aesthetic correction and skin revitalization are appreciated.

The volumetric defects were filled, with a very natural final appearance. Furthermore, on the duration in months, the decay of the presence of the hydrogel is very physiological and slow, an advantage which is associated with the lack of immediate and continued pain in the injection site.

These aspects must be ascribed, at least in part, to the particular preparation procedure of the cross-linked hyaluronic acid and to the adsorption of the two amino acids glycine and proline which are released for a long time.

The presence of the two amino acids glycine and proline, adsorbed in the cross-linked hyaluronic acid with the innovative procedure according to the invention, released in a time span of 50-60 hours, confers an autonomous improvement for the actions performed by these amino acids, certainly with a longer effect than a similar formulation containing only non cross-linked hyaluronic acid and amino acids which, due to the structure of the non cross-linked hyaluronic acid, are not adsorbed and therefore are released in certainly shorter times.

### FINAL CONCLUSION.

The cross-linked hyaluronic acid containing amino acids specifically adsorbed in the macrostructure thereof, developed by the company applying for the patent in hand, proved to be considered, due to the peculiarity thereof of releasing the two amino acids glycine and proline over time, specifically adsorbed therein, and due to the effects shown in vivo, as an excellent long-lasting filler with a good ability to stimulate collagen.

## Claims

1. A method for producing a cross-linked hyaluronic acid in the form of an injectable gel, sterile by sterilization at 121°C, wherein two amino acids such as glycine and proline are adsorbed, having the ability to modulate the release thereof, allowing a stimulation of collagen synthesis for a long time, **characterized by** providing that said amino acids are adsorbed in the hyaluronic acid gel after the cross-linking reaction.

2. A method for producing a cross-linked hyaluronic acid in the form of an injectable gel, sterile by sterilization at 121°C, wherein two amino acids such as glycine and proline are adsorbed, having the ability to modulate the release thereof, allowing a stimulation of collagen synthesis for a long time, according to the preceding claim, **characterized by** including the following steps in sequence:
I) SOLUBILIZATION OF HYALURONIC ACID
a 10% non-linked hyaluronic acid is solubilized in a 1 N NaOH solution; the polymerizing agent BDDE is added at 1% by weight with respect to the hyaluronic acid, and after homogenization and incubation at a controlled temperature,
II) GEL NEUTRALIZATION
the homogenized gel is neutralized and incubated with a 1% by weight phosphoric acid solution;
III) STEP OF AMINO ACID ABSORPTION IN THE CROSS-LINKED HYALURONIC ACID
a phosphate buffer solution containing glycine and proline is added to the neutralized cross-linked gel in an amount such that the final concentration of each of the two amino acids in the 2.4% cross-linked hyaluronic acid gel is between 1% and 2.5% by weight.
V) ABSORPTION MODE
the adsorption of said amino acids is carried out in the cross-linked gel by swelling it for 7 days at room temperature under slight mixing until a compact and fluid gel is obtained where the previously added buffer solution containing amino acids is completely adsorbed and
V) FLUENT STEAM STERILIZATION
the gel obtained is sterilized, divided into glass syringes, through sterilization at 121°C for 15 minutes.

3. A method for producing a cross-linked hyaluronic acid in the form of an injectable gel, wherein two amino acids such as glycine and proline are adsorbed, according to the preceding claim, **characterized in that** the gel incubation occurs during the cross-linking reaction by placing the gel in an oven for 4 hours at 40°C.

4. A method according to claim 1, **characterized in that** by varying the final concentration of the two amino acids glycine and proline in a range between 2.5% and 1% by weight in the neutralized gel of 2.4% cross-linked hyaluronic acid by weight, the release times of the same amino acids glycine and proline, once adsorbed, remain unaltered.

5. A locally injectable cross-linked hyaluronic acid gel, with prolonged release over time of glycine and proline useful for the stimulation of collagen synthesis obtained according to each of claims 1 to 3, or a combination thereof, for cosmetic use.

6. A locally injectable cross-linked hyaluronic acid gel with prolonged release of glycine and proline for the stimulation of collagen synthesis, sterile by sterilization at 121°C, where the final concentration of both the two amino acids in the 2.4% hyaluronic acid gel (24mg/ml) is 2.5% (25 mg/ml).

7. A cross-linked hyaluronic acid gel according to the preceding claim, **characterized in that** with the same concentration of 2.4% by weight of cross-linked hyaluronic acid, the release over time of the two adsorbed amino acids glycine and proline is not modified if both the concentrations thereof switch from 2.5% by weight to a concentration of 1% by weight.

8. A cross-linked hyaluronic acid gel according to any one of claims 3 to 5, **characterized in that** it is marketed in single-dose glass syringes after sterilization.

9. A hyaluronic acid gel according to claim 3 or 4, to be used as a long-lasting filler for cosmetic use.
